(19) Europäisches Patentamt
European Patent Office
Office européen des brevets

(11) Publication number: **0 304 731 A1**

# (12) EUROPEAN PATENT APPLICATION

(21) Application number: 88113053.8

(51) Int. Cl.[4]: **C07D 211/56**

(22) Date of filing: 11.08.88

(30) Priority: 28.08.87 JP 215812/87

(43) Date of publication of application: 01.03.89 Bulletin 89/09

(84) Designated Contracting States: DE FR GB IT

(71) Applicant: NIPPON SHINYAKU CO., LTD.
14, Kisshoinnishinoshomonguchicho
Minami-ku Kyoto-shi Kyoto 601(JP)

(72) Inventor: Hasegawa, Akira
1735-160, Kano
Gifu-shi Gifu-ken(JP)
Inventor: Kiso, Makoto
57-47 Motosucho Monju
Motosu-gun Gifu-ken(JP)
Inventor: Yoshikuni, Yoshiaki
4-101 Yuni-Ujigawa-Manshon 33
Kobatanishinaka
Uji-shi Kyoto-fu(JP)

(74) Representative: Kügele, Bernhard et al
c/o NOVAPAT-CABINET CHEREAU 9, Rue du Valais
CH-1202 Genève(CH)

## (54) Moranoline derivatives.

(57) Compounds represented by the following general formula (I):

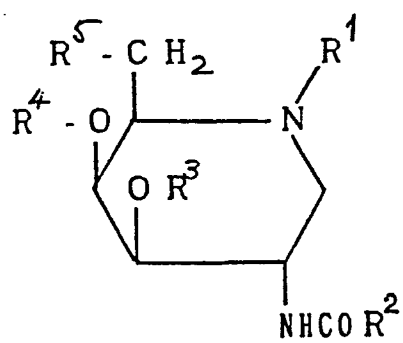

( I )

wherein $R^1$ is hydrogen or alkyloxycarbonyl; $R^2$ is lower alkyl; $R^3$ is hydrogen or acyl; $R^4$ is hydrogen or acyl; and $R^5$ is hydroxy or triarylmethyloxy. These compounds are derivatives of moranoline and exhibit useful pharamacological activities such as hyaluronidase inhibition, N-acetylgalactosaminidase inhibition, etc.

EP 0 304 731 A1

# MORANOLINE DERIVATIVES

Detailed Description of the Invention:

(Technical Field)

The present invention relates to novel compounds which are derivatives of moranoline and exhibit useful pharmacological activities such as hyaluronidase inhibition, N-acetylgalactosaminidase inhibition, etc.

The compounds of the present invention are represented by the following general formula (I):

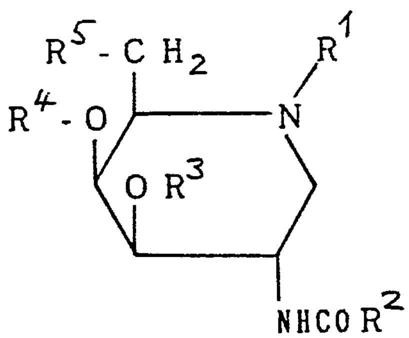

( I )

wherein $R^1$ is hydrogen or alkyloxycarbonyl; $R^2$ is lower alkyl; $R^3$ is hydrogen or acyl; $R^4$ is hydrogen or acyl; and $R^5$ is hydroxy or triarylmethyloxy.

(Prior Art)

Since moranoline has been found to exhibit good blood glucose decreasing activity in 1976, various derivatives have been synthesized by the present inventors and studies have been continued (cf. Nippon Nogei Kagaku Kaishi, 50, 571, 1976 and others including many patent applications).

(Problems to be solved by the Invention)

The present inventors have conducted further studies with a purpose of testing physiological activity of those compounds and have confirmed that compounds having certain steric structure easily connect with enzymes having an important role in vivo because of the steric specificity.

Accordingly an object of the present invention is to afford compounds having novel pharmacological effect from the fact that certain group of compounds having steric specificity among moranoline derivatives inhibits certain enzymatic activity in vivo.

(Means to solve the Problems)

The characteristic feature of the present invention is that the present invention compound having the above-given general formula (I) exhibits certain fundamental structure with specific steric configuration.

The characteristic point of the compound represented by the general formula (I) is in the steric configuration of fundamental skeleton of its sugar moiety. Moranoline and derivatives thereof have been characterized in having glucose type. However, the present inventors have changed it to the steric configuration which is same as galactose (hereinafter called "galacto type") and the interrelationship between the steric configuration and physiological activity has been studies whereupon the following fact has been found and the present invention has been achieved.

In the compounds of the present invention, the term "galacto type" stands for that (1) 2-position of the moranoline skeleton is in an alpha-configuration; (2) 3-position of it is in a beta-configuration; (3) 4-position is in a beta-configuration; and (4) 5-position is in a beta-configuration. As a result of such a steric configuration structure of the present invention compund, the effect which is characteristic to the present invention is achieved.

$R^1$ in the general formula (I) may be alkyloxycarbonyl besides hydrogen and alkyl and is, for example, methoxycarbonyl, ethoxycarbonyl, propoxycarbonyl, butoxycarbonyl, pentoxycarbonyl, etc. and, among those, tert-butoxycarbonyl (Boc) is particularly preferred in terms of an object of the present invention.

Examples of $R^2$ are lower alkyl and the carbon atoms in the alkyl are preferably from 1 to 4.

Examples of $R^3$ are acyl such as acetyl besides hydrogen.

Examples of $R^4$ are acyl scuh as acetyl besides hydrogen.

Examples of $R^5$ are triarylmethyloxy such as triphenylmethyloxy besides hydroxy.

As a result of its specific steric configuration, the present invention compound exhibits inhibitnig action on hyaluronidase. Accordingly, the present invention compound can be used, for example, as antiallergic drug and is useful as a pharmaceutical agent.

Moreover, the present invention compound exhibits inhibitory action against N-acetylgalactosaminidase. Furthermore, it exhibits antibacterial activity.

In view of the above, the present invention compound is applicable as, for example, antiviral, antibacterial, and cancer-metastase inhibiting agent and is quite useful as medicament.

It has been known that toxicity of the present invention compound is very low.

When the present invention compound is administered as a drug, it is given, without or with pharmaceutically-acceptable nontoxic and inert carrier as a pharmaceutical composition containing 0.1 to 99.5%, preferably 0.5 to 90%, of the compound, to animals including human being.

As to a carrier, one or more auxiliary agents for pharmaceutical prescription in a form of solid, semisolid, or liquid including diluent and filler may be used. It is preferred that the pharmaceutical composition is given in a unit dose form. The pharmaceutical composition of the present invention may be administered via mouth, to tissue, to local place (e.g. via skin) or via rectum. Needless to say, pharmaceutical dosage form suitable for each administration route is adopted.

It is desired that the dose as antiallergic drug is decided taking the state of the patient (age, body weight, etc.), administration route and type and degree of the disease into consideration. However, usually, the dose of the effective ingredient is 100 mg to 3 g/day, or preferably 500 mg to 1 g/day, for human being. In some cases, smaller dose may be sufficient while, in other cases, more dose may be necessary. It is desired that the administration is conducted dividedly, i.e. 1 to 3 times a day.

Compound of the present invention may, for example, be prepared by the following manner.

First, the position of N of the moranoline structure which is a fundamental skeleton of the present invention compound is protected with a suitable protective group. 4- and 6-positions of the moranoline can be protected by, for example, introduction of benzylidene group there-into so that a ring is formed. Similarly, 2- and 3-positions can be protected by the reaction with azide followed by, for example, acetyl. Then a benzylidene group which forms a ring at 4- and 6-positions is removed.

Then triphenylmethyl group, for example, is introduced whereupon it can be introdued in 6-position selectively because said group is bulky whereupon the compound in which only 4-position is unprotected is resulted.

This compound is then oxidized whereupon the free hydroxy group at 4-position is changed to ketone. Then it is reduced again so that the 4-position which was in an alpha-configuration can be easily covered to a beta-configuration. By converting the alpha-configuration of the 4-position to the beta-configuration in the moranoline skeleton of glucose type, the galacto type of the present invention can be resulted. Later, the protective groups can be removed if and when necessary.

(Examples)

The present invention will be further illustrated by way of giving examples concerning the manufacture of the present invention compounds as hereinafter.

Reférence Example.

(1) Moranoline (2.0 g) was dissolved in 1 ml of water, 1.6 ml of triethylamine and a solution of 11.6 g of 2-tert-butoxycarbonylthio-4,6-dimethylpyrimidine in 1 ml of dioxane were added, and the mixture was stirred overnight at 60° C. Completion of the reaction was confirmed by thin layer chromatography, then reaction solution was lyophilyzed to remove water and dioxane, the resulting residue was subjected to a column chromatography (Wakogel C-200 being used; elutes used were a mixture of $CH_2Cl_2$ and $CH_3OH$ in a ratio of 100:1 (a), 50:1 (b) or 20:1 (c)) and from the eluate with the (c) was obtained 3.16 g of N-tert-butoxycarbonyl-1,5-dideoxy-1,5-imino-D-glucitol.

(2) The above-resulted compound (3.2 g) was dissolved in 30 ml of N,N-dimethylformamide, an excess of Drierite was added, the mixture was stirred at room temperature for 1 hour, then 5.5 ml of benzaldehyde dimethylacetal and a catalytic amount of p-toluenesulfonic acid were added, and the mixture was stirred at room temperature for 10 hours. After completion of the reaction, methanol was added thereto, neutralized with Amberlite IR 410 ($OH^-$), and filtered. After well washing with methanol, the filtrate was combined with the washing, and the mixture was concentrated in vacuo at 45° C. The resulting was subjected to a column chromatography (Wakogel C-200 being used: the eluting solutions were a mixture of $CH_2Cl_2$ nd $CH_3OH$ in a ratio of 1:0 (a) and 100:1 (b)). The oil obtained from the eluate with (b) was crystallized from ethyl acetate-n-hexane to give 2.9 g( 67%) of 4,6-O-benzylidene-N-tert-butoxycarbonyl-1,5-dideoxy-1,5-imino-D-glucitol.

(3) The product (1.0 g) obtained in the above (2) was dissolved in 25 ml of dry methylene chloride, then 3.6 ml of 2,6-lutidine, 0.5 ml of triethylamine and 0.58 g of chloroacetic anhydride were added, and the mixture was stirred at 0° C for

1 hour. After confirming the completion of the reaction by thin layer chromatography, the reaction solution was extracted with methylene chloride (3 times 30 ml) and washed with 2N hydrochloric acid and water. The extracted layer was dried over sodium sulfate, filtered, well washed with methylene chloride, the washing was combined with the filtrate, the mixture was concentrated in vacuo at 45° C, the resulting residue was subjected to a column chromatography (Wakogel C-200 being used: the eluting solutions used were $CH_2Cl_2$:$CH_3OH$ in a ratio of 1:0 (a), 400:1 (b), 300:1 (c) and 100:1 (d)) and, from the eluates with (b) and (C), 300 mg (24.6%) of 4,6-O-benzylidene-N-tert-butoxycarbonyl-2-O-chloroacetyl-1,5-dideoxy-1,5-imino-D-glucitol and 600 mg (49.3%) of 4,6-O-benzylidene-N-tert-butoxycarbonyl-3-O-chloroacetyl-1,5-dideoxy-1,5-imino-D-glucitol, respectively.

(4) The above 4,6-O-benzylidene-N-tert-butoxycarbonyl-3-O-chloroacetyl-1,5-dideoxy-1,5-imino-D-glucitol (610 mg) was dissolved in 3 ml of dry pyridine, 0.21 ml of methane sulfonyl chloride was added thereto at -20° C and, with a gradual turn to room temperature, the mixture was stirred for 5 hours. After confirming the completion of the reaction by thin layer chromatography, the reaction solution was extracted with methylene chloride (3 times 30 ml) and washed with 2N hydrochloric acid and water. The extracted layer was dried with sodium sulfate, filtered and well washed with methylene chloride. The filtrate was combined with the washing and the mixture was concentrated in vacuo to give 770 mg of 4,6-O-benzylidene-N-tert-butoxycarbonyl-3-O-chloroacetyl-1,5-dideoxy-1,5-imino-2-O-mesyl-D-glucitol.

(5) The compound ( 1.5 g) obtained in the above (4) was dissolved in a mixture of 0.5 ml of dry dioxane and 2 ml of dry methanol, then 3 ml of 28% sodium methoxide solution was added thereto, and the mixture was stirred for 2 hours at room temperature. After confirming the completion of the reaction by a thin layer chromatography, the reaction solution was concentrated in vacuo at room temperature, and extracted with methylene chloride (3 times 50 ml). The extracted layer was dried with sodium sulfate, filtered, well washed with methylene chloride. The filtrate was combined with the washing and concentrated in vacuo to give 1.02 g of 2,3-anhydro-4,6-O-benzylidene-N-tert-butoxycarbonyl-1,5-dideoxy-1,5-imino-D-mannitol.

(6) The compound (40 mg) obtained in the above (5) was dissolved in 1 ml of N,N-dimethylformamide, 90 mg of sodium azide was added, and the mixture was made to react at 110° C for 6 hours. After confirming the completion of the reaction by thin layer chromatography, the reaction solution was concentrated in vacuo at 60° C, the resulting residue was dissolved in 30 ml of methylene chloride, and washed with water. The methylene chloride layer was dried with sodium sulfate, filtered, and well washed with methylene chloride. The filtrate was combined with the washing, the mixture was concentrated in vacuo at 45° C and the resulting residue was subjected to a column chromatography (Wakogel C-200 being used; eluting solution used was $CH_2Cl_2$) whereupon separation/purification was conducted giving 25.6 mg (59%) of 2-azido-4,6-O-benzylidene-N-tert-butoxycarbonyl-1,5-imino-1,2,5-trideoxy-D-glucitol.

(7) The compound (1.4 g) obtained in the above (6) was dissolved in 10 ml of methanol, 1.3 g of ammonium formate and 2 g of 10% Pd-C which was previously washed with methanol and ethanol were added and the mixture was stirred for 10 minutes at room temperature. After confirming the completion of the reaction by thin layer chromatograhy, 10% Pd-C was removed by filtration, and washed with methanol. The filtrate was combined with the washing, the mixture was concentrated in vacuo, to the resulting solid were added 10 ml of pyridine and 10 ml of acetic anhydride, and the mixture was stirred for 2 hours at room temperature. After confirming the comple tion of the reaction by thin layer chromatography, 15 ml of methanol was added thereto at 0° C and the mixture was further concentrated in vacuo. The resulting residue was extracted with methylene chloride, washed with 2N hydrochloric acid and water, dried with sodium sulfate, and concentrated in vacuo. The resulting residue was crystallized from ether to give 1.44 g (89%) of 2-acetamido-3-O-acetyl-4,6-O-benzylidene-N-tert-butoxycarbonyl-1,2,5-trideoxy-1,5-imino-D-glucitol.

(8) The compound (1.4 g) obtained in the above (7) was dissolved in 10 ml of acetic acid and 5 ml of methanol, 10% Pd-C which was previously washed was added thereto, and subjected to hydrogenation for 2 days at room temperature. After confirming the completion of the reaction by thin layer chromatography, 10% Pd-C was filtered off, and washed with methanol. The filtrate was combined with the washing, the mixture was concentrated in vacuo, the resulting residue was subjected to a column chromatography (Wakogel C-200 being used: the eluting solutions used were $CH_2Cl_2$:$CH_3OH$ in 100:1 (a) and 20:1 (b)), and from the eluate with (b) was obtained 650 mg (60%) Of 2-acetamido-3-O-acetyl-N-tert-butoxycarbonyl-1,2,5-trideoxy-1,5-imino-D-glucitol.

(9) The compound (60 mg) obtained in the above (8) was dissolved in 5 ml of pyridine, 100 mg of trityl chloride was added, and stirred overnight at 60° C. After confirming the completion of the reaction by thin layer chromatography, the reaction solution was extracted with methylene chloride, and washed with 2N hydrochloric acid and water. This was dried with sodium sulfate, concentrated in vacuo, the resulting residue was subjected to a column chromatography (Wakogel C-200 being used; eluting solutions were $CH_2Cl_2$: $CH_3OH$ in 1:0 (a) and 50:1 (b)) and, from the eluate with (b), 93 mg (89%) of 2-acetamido-3-O-acetyl-N-tert-butoxycarbonyl-1,2,5-trideoxy-1,5-imino-6-O-triphenylmethyl-D-glucitol was obtained.

(10) To 5 ml of methylene chloride and 100 mg of pyridine was added 130 mg of chromium oxide, the mixture was stirred for 1 hour at 0°C, a solution of 190 mg of a compound obtained in the above (9) dissolved in 2 ml of methylene chloride was added, and the mixture was stirred 1.5 hours at 0°C. After confirming the completion of the reaction by thin layer chromatography, the reaction solution was subjected to a column chromatography (Wakogel C-200 being used; the eluting solution was AcOEt) to give 175 mg (92%) of 2-acetamido-3-O-acetyl-N-tert-butoxycarbonyl-4-dehydro-1,2,5-trideoxy-1,5-imino-6-O-triphenylmethyl-D-glucitol. This was crystallized from ethanol.

Example 1. Synthesis of 2-acetamido-3-O-acetyl-N-tert-butoxycarbonyl-1,2,5-trideoxy-1,5-imino-6-O-triphenylmethyl-D-galactitol.

2-Acetamido-3-O-acetyl-N-tert-butoxycarbonyl-4-dehydro-1,2,5-trideoxy-1,5-imino-6-O-triphenylmethyl-D-glucitol (140 mg) was dissolved in 3 ml of methanol and 0.5 ml of ethanol, then 40 mg of $NaBH_4$ was added at 0°C, and the mixture was stirred for 30 minutes. After confirming the completion of the reaction by thin layer chromatography, the reaction solution was neutralized with Amberlite IR 120 ($H^+$), and concentrated in vacuo. The resulting residue was subjected to a column chromatography (Wakogel C-200 being used; the eluting solutions used were $CH_2Cl_2$:$CH_3OH$ in 150:1 (a), 100:1 (b) and 50:1 (c)) and from the eluate (c) was obtained 86 mg of the desired compound (yield: 61%).

$[\alpha]_D$ = -17.7° (c = 3.02, $CH_2Cl_2$).

IR $\nu_{max}^{film}$ : $cm^{-1}$: 3350 (OH, NH), 2990, 2940, 2870 (Me, $CH_2$), 1740 (CO in Ac), 1660 (CO in Boc), 1640, 1540 (amide), 700 (phenyl).

Example 2. Syntheses of (1) 2-acetamido-3-O-acetyl-N-tert-butoxycarbonyl-1,2,5-trideoxy-1,5-imino-D-galactitol and (2) 2-acetamido-4-O-acetyl-N-tert-butoxycarbonyl-1,2,5-trideoxy-1,5-imino-D-galactitol.

2-Acetamido-3-O-acetyl-N-tert-butoxycarbonyl-1,2,5-trideoxy-1,5-imino-6-O-triphenylmethyl-D-galactitol (270 mg) was dissolved in 10 ml of acetic acid and 5 ml of methanol, 150 mg of 10% Pd-C which was previously dissolved in ethanol and methanol was added, and subjected to hydrogenation for 3 days at room temperature. After confirming the completion of the reaction by thin layer chromatography, 10% Pd-C was filtered off, and washed with methanol. The filtrate was combined with the washing, the mixture was concentrated in vacuo, the resulting residue was subjected to a column chromatography (Wakogel C-200 being used; the eluting solutions used were $CH_2Cl_2$:$CH_3OH$ in 50:1 (a), 40:1 (b), 20:1 (c) and 10:1 (d)), and from the eluate with (d) was obtained 50 mg (yield: 32%) of the desired compound (1) while from the eluate with (c) was obtained 70 mg (45%) of the desired compound (2).

$[\alpha]_D$ = -38.2° (c = 0.90, methanol) for (1)
= +10.2° (c = 1.21, methanol) for (2).

M.p. (1): amorphous
(2): 157 to 159°C

IR $\nu_{max}^{film}$ ($cm^{-1}$):
(1): 3350 (NH, OH), 2950, 2860 (Me, $CH_2$), 1740 (CO in Ac), 1670 (CO in Boc), 1640, 1530 (amide), (complete disappearance of 700 (phenyl));
(2): 3350 (NH, OH), 2970, 2860 (Me, $CH_2$), 1740 (CO in Ac), 1660 (CO in Boc), 1640, 1530 (amide), (complete disappearance of 700 (phenyl)).

NMR (270 MHz, $CD_3OD$):
(1) $\delta$ : 1.50 (s, 9H, 3Me in Boc); 2.13 (s, 3H, AcN); 2.17 (s, 3H, AcO); 5.04 (dd, 1H, $J_{3.2}$ = 9.89 Hz, $J_{3.4}$ = 1.46 Hz H-3) complete disappearance of 7.2-7.4 (m, 15H, Tr-3 x Ph);
(2) $\delta$ : 1.50 (s, 9H, 3Me in Boc); 2.05 (s, 3H, AcN); 2.08 (s, 3H, AcO); complete disappearance of 7.2-7.4 (m, 15H, Tr-3 x Ph).

Example 3. Synthesis of 2-acetamido-N-tert-butoxycarbonyl-1,2,5-trideoxy-1,5-imino-D-galactitol.

2-Acetamido-3-O-acetyl-N-tert-butoxycarbonyl-1,2,5-trideoxy-1,5-imino-D-galactitol (48 mg) was dissolved in 2 ml of methanol, 0.5 ml of 28% NaOMe was added at 0°C, and stirred for 20 minutes. After confirming the completion of the reaction by thin layer chromatography, the reaction solution was neutralized with Amberlite IR-120 ($H^+$). The resin was filtered off and washed with methanol. The filtrate was combined with the washing and concentrated in vacuo to give 42.7 mg (96%) of the desired compound.

$[\alpha]_D$ = -1.87° (c = 0.85, $CH_3OH$).

M.P.: 156-160°C.

IR $\nu_{max}^{film}$ ($cm^{-1}$): 3400 (NH, OH), 2960, 2880 (Me, $CH_2$), 1670 (CO in Boc), 1640, 1540

Example 3. Synthesis of 2-acetamido-N-tert-butoxycarbonyl-1,2,5-trideoxy-1,5-imino-D-galactitol.

2-Acetamido-3-O-acetyl-N-tert-butoxycarbonyl-1,2,5-trideoxy-1,5-imino-D-galactitol (48 mg) was dissolved in 2 ml of methanol, 0.5 ml of 28% NaOMe was added at 0°C, and stirred for 20 minutes. After confirming the completion of the reaction by thin layer chromatography, the reaction solution was neutralized with Amberlite IR-120 ($H^+$). The resin was filtered off and washed with methanol. The filtrate was combined with the washing and concentrated in vacuo to give 42.7 mg (96%) of the desired compound.

$[\alpha]_D$ = -1.87° (c = 0.85, $CH_3OH$).

M.p.: 156-160°C.

IR $\nu_{max}^{film}$ ($cm^{-1}$): 3400 (NH, OH), 2960, 2880 (Me, $CH_2$), 1670 (CO in Boc), 1640, 1540 (amide), (complete disappearance of 1740 (Ac)).

NMR: (270 MHz, $CD_3OD$)

$\delta$: 1.50 (s, 9H, 3Me in Boc); 2.07 (s, 3H, AcN); (complete disappearance of 2.17 (Ac)).

Example 4. Synthesis of 2-acetamido-1,2,5-trideoxy-1,5-imino-D-galactitol.

To 140 mg of 2-acetamido-N-tert-butoxycarbonyl-1,2,5-trideoxy-1,5-imino-D-galactitol was added 5 ml of trifluoroacetic acid at 0°C and the mixture was stirred for 1 hour at room temperature. The reaction solution was concentrated in vacuo, ether was added thereto, and decantated. The resulting

solid was dissolved in 10 ml of methanol and the solution was stirred for 30 minutes with Amberlite IR-410 ($OH^-$). The resin was filtered off and concentrated in vacuo to give 94 mg(97%) of the desired product.

$[\alpha]_D$ = +2.0° (c = 0.94, $CH_3OH$).

M.p. (hygroscopic)

IR $\nu_{max}^{Nujol}$ ($cm^{-1}$): 3400 (NH, OH), 2960, 2900 (Me, $CH_2$), 1650, 1560 (amide), (complete disappearance of 1670 (CO in Boc)).

( End )

CLAIM

A compound represented by the following general formula (I):

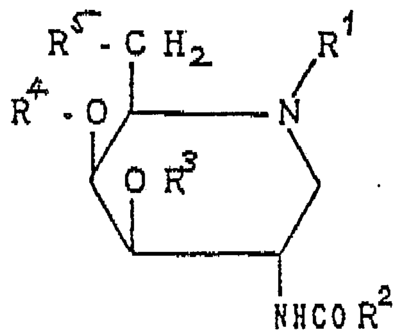

(I)

wherein $R^1$ is hydrogen or alkyloxycarbonyl; $R^2$ is lower alkyl; $R^3$ is hydrogen or acyl; $R^4$ is hydrogen or acyl; and $R^5$ is hydroxy or triarylmethyloxy.

European Patent Office

# EUROPEAN SEARCH REPORT

Application number

EP 88113053.8

**DOCUMENTS CONSIDERED TO BE RELEVANT**

| Category | Citation of document with indication, where appropriate, of relevant passages | Relevant to claim | CLASSIFICATION OF THE APPLICATION (Int. Cl.4) |
|---|---|---|---|
| A | EP - A2/A3 - 0 230 581 (BAYER AG)<br>* Abstract *<br>-- | 1 | C 07 D 211/56 |
| A | GB - A - 2 020 278 (NIPPON SHINYAKU)<br>* Abstract *<br>---- | 1 | |
| | | | TECHNICAL FIELDS SEARCHED (Int. Cl.4) |
| | | | C 07 D 211/00 |

The present search report has been drawn up for all claims

| Place of search | Date of completion of the search | Examiner |
|---|---|---|
| VIENNA | 29-11-1988 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82